(19) Europäisches Patentamt
European Patent Office
Office européen des brevets

(11) **EP 1 192 942 B1**

(12) **EUROPEAN PATENT SPECIFICATION**

(45) Date of publication and mention
of the grant of the patent:
**25.07.2007 Bulletin 2007/30**

(51) Int Cl.:
**A61K 9/20** (2006.01)     **C08B 11/20** (2006.01)

(21) Application number: **01307729.2**

(22) Date of filing: **11.09.2001**

(54) **Base material for dry direct tableting comprising low-substituted hydroxypropyl cellulose**

Basismaterial zur trockenen Direkttablettierung enthaltend niedrig substituierte Hydroxypropylcellulose

Matériau de base pour la compression directe sèche contenant une hydoxypropylcellulose à faible substitution

(84) Designated Contracting States:
**CH DE FR GB LI**

(30) Priority: **27.09.2000 JP 2000293279**

(43) Date of publication of application:
**03.04.2002 Bulletin 2002/14**

(73) Proprietor: **Shin-Etsu Chemical Co., Ltd.**
**Chiyoda-ku,**
**Tokyo (JP)**

(72) Inventor: **Maruyama, Naosuke,**
**c/o Specialty Chem. Res. Center**
**Kubiki-mura**
**Naka Kubiki-gun,**
**Niigata-ken (JP)**

(74) Representative: **Goddard, David John et al**
**Harrison Goddard Foote**
**Orlando House**
**11c Compstall Road**
**Marple Bridge**
**Stockport SK6 5HH (GB)**

(56) References cited:
EP-A- 0 957 112          EP-A- 1 054 019
EP-A- 1 099 709          EP-A- 1 133 984
EP-A- 1 285 655          WO-A-98/53798
US-A- 4 454 108          US-A- 5 200 194

• ZUURMAN K ET AL: "Effect of binder on the relationship between bulk density and compactibility of lactose granulations." INTERNATIONAL JOURNAL OF PHARMACEUTICS (AMSTERDAM), vol. 119, no. 1, 1995, pages 65-69, XP002224192 ISSN: 0378-5173

Remarks:
The file contains technical information submitted after the application was filed and not included in this specification

**Description**

BACKGROUND OF THE INVENTION

1. Field of the invention

This invention generally relates to a method of manufacturing a low-substituted hydroxypropyl cellulose that is added for the purpose of imparting disintegration properties or binding properties during the manufacture of preparations in the fields of medicines, foods and the like.

2. Description of the related art

In solid preparations for use in the fields of medicines, foods and the like, those composed of principal components alone have problems in that, when they are administered as medicines, they may not be satisfactorily disintegrated to such an extent as to exhibit a sufficient drug effect or in that, when they are formed into tablets or granules, they may fail to retain their form owing to poor binding properties. In such cases, disintegration properties or binding properties can be imparted by adding low-substituted hydroxypropyl cellulose to preparations.

Besides low-substituted hydroxypropyl cellulose, additives used for this purpose include carboxymethylcellulose calcium, crosslinked carboxymethylcellulose sodium, crosslinked polyvinyl pyrrolidone, carboxymethyl starch and the like. However, low-substituted hydroxypropyl cellulose has the advantage that it is nonionic and hence less liable to changes in properties due to reaction with ionic drugs or the like.

This advantage is utilized, for example, in a process wherein a powder of low-substituted hydroxypropyl cellulose is dry-blended with a drug and other ingredients (e.g., excipients), and the resulting blend is formed into tablets; and in a process wherein a powder of low-substituted hydroxypropyl cellulose is granulated by kneading it with water or an aqueous solution of a water-soluble binder, and the resulting granules are molded. This low-substituted hydroxypropyl cellulose is a pharmaceutical additive described in the Pharmacopoeia of Japan, and its use as a pharmaceutical additive is disclosed in Japanese Patent Publication Nos. 46-42792/'71 and 57-53100/'82.

EP-A-0 957 112, which is considered to be the closest prior art, discloses low-substituted hydroxypropyl cellulose having a loose bulk density of not less than 0.40 g/ml and a tap bulk density of not less than 0.60 g/mL and a process for producing the same.

Low-substituted hydroxypropyl cellulose may be produced as the reaction product of an alkali cellulose with propylene oxide. This can be done, for example, by soaking pulp in an aqueous solution of sodium hydroxide, pressing it to yield an alkali cellulose, and reacting the alkali cellulose with propylene oxide, or by dispersing powdered pulp in an organic solvent (e.g., isopropyl alcohol, tert-butyl alcohol or hexane), adding an aqueous solution of sodium hydroxide thereto so as to yield an alkali cellulose, and adding propylene oxide thereto and reacting it therewith.

Low-substituted hydroxypropyl cellulose is soluble in aqueous alkaline solutions, and sodium hydroxide used as a catalyst remains in the reaction product. This reaction product is dissolved in water, and the remaining alkali is neutralized with an acid to form neutralization-precipitated particles of low-substituted hydroxypropyl cellulose.

In order to remove the salt formed in this step and other impurities, the neutralization-precipitated particles are washed with water or hot water. The washed material is pressed to remove water, dried, and pulverized to yield a final product of low-substituted hydroxypropyl cellulose.

This low-substituted hydroxypropyl cellulose is in the form of a powder comprising a mixture of a fibrous material and a spherical material. It is said that, when it is used to form tablets and the like, its binding properties are created by interlocking of this fibrous material. On the other hand, if the proportion of this fibrous material is increased in order to enhance binding properties, the resulting powder becomes bulky and hence shows a reduction in flowability. Consequently, in a process wherein a powder of low-substituted hydroxypropyl cellulose is dry-blended with a drug and other ingredients (e.g., excipients) and the resulting blend is formed into tablets(i.e., a process commonly called "dry direct tableting"), this low flowability is problematic in that the formation of tablets may be impossible because the blend fails to flow out of the hopper of a tableting machine or in that there may be a wide variation in the weight of tablets. Japanese Patent Provisional Publication No. 7-324101/'95 discloses a certain type of low-substituted hydroxypropyl cellulose characterized by an angle of repose of 45 degree or less and a degree of swelling of 100% or greater. Although this low-substituted hydroxypropyl cellulose shows a slight improvement in flowability, it has the disadvantage that a decrease of the fibrous material causes a reduction in binding properties.

SUMMARY OF THE INVENTION

The present invention has been made in view of the above-described circumstances, and an object thereof is to modify low-substituted hydroxypropyl cellulose added as a binder and disintegrator in the formation of tablets, so as to serve as a base material for dry direct tableting having high binding power and good flowability.

As a result of intensive investigations carried out with a view to accomplishing the above object, the present inventors have now found that a product obtained by impregnating low-substituted hydroxypropyl cellulose with a sugar or a sugar alcohol serves as a base material for dry direct tableting showing an improvement in binding power and flowability. The present invention has been completed on the basis of this finding.

Thus, the present invention provides a method of manufacturing a base material for dry direct tableting showing an improvement in binding power and flowability, the base material being obtained by impregnating low-substituted hydroxypropyl cellulose with a sugar or a sugar alcohol and then drying it, according to claim 1 or 2.

The product obtained by impregnating low-substituted hydroxypropyl cellulose with a sugar or a sugar alcohol and then drying it serves as a binder and disintegrator having high binding power and good disintegrability, and can hence be utilized as a base material for dry direct tableting.

## DETAILED DESCRIPTION OF PREFERRED EMBODIMENTS

The present invention will be more specifically described hereinbelow.

As used herein, the term "base material for dry direct tableting" means any of various excipients, binders and disintegrators other than drugs, which are used in the formation of tablets and the like in a dry process. The term "dry direct tableting" means a process in which a drug is blended with an excipient, a binder and/or a disintegrator in powder form without using water or other solvent, and the resulting blend is compression-molded with a tableting machine to form tablets or the like. This process has the advantage that it is highly simplified because materials in powder form are directly blended and compression-molded.

The low-substituted hydroxypropyl cellulose of the present invention has hydroxypropoxyl content in the range of 5.0 to 16.0% by weight. Its hydroxypropoxyl content can be determined by according to the method of quantitative determination described in Japanese Pharmacopoeia under the head of "Low-substituted Hydroxypropyl Cellulose". In the low-substituted hydroxypropyl cellulose used in the present invention, the number of moles of hydroxypropoxyl substituent group per mole of anhydrous glucose unit ($C_6H_{10}O_5$) is preferably in the range of 0.1 to 0.5. If the number of moles of hydroxypropoxyl substituent group is less than 0.1, the resulting product may not show the desired binding properties. If it is greater than 0.5, the resulting product may not show the desired disintegration properties and, therefore, the resulting preparations (e.g., tablets) may have an unduly long disintegration time.

The sugar or sugar alcohol used in the present invention comprises one or more compounds selected from the group consisting of erythritol, mannitol, sorbitol, lactose, sucrose and the like. However, sugar alcohols having no reducing terminal, such as erythritol, mannitol and sorbitol, are preferred because of their good shelf stability.

According to the process for preparing this base material for dry direct tableting obtained by impregnating low-substituted hydroxypropyl cellulose with a sugar or a sugar alcohol and then drying it, a wet granular material is prepared by dry-blending low-substituted hydroxypropyl cellulose with a sugar or a sugar alcohol and then adding 200 to 600% by weight of water to the resulting blend while agitating it, or by adding an aqueous solution of a sugar or a sugar alcohol to low-substituted hydroxypropyl cellulose while agitating it , wherein 30-100% by weight sugar or sugar alcohol is added, based on said low-substituted hydroxypropyl cellulose. Thereafter, the resulting granular material is dried in the usual manner, and may be pulverized and classified as required. Thus, the desired base material for dry direct tableting comprising low-substituted hydroxypropyl cellulose impregnated with a sugar or a sugar alcohol can be obtained.

No particular limitation is placed on the type of low-substituted hydroxypropyl cellulose used for this purpose. However, in order to enhance binding power, it is preferable to use low-substituted hydroxypropyl cellulose in fibrous form. Degree of compaction serves as an index thereto, and low-substituted hydroxypropyl cellulose preferably has a degree of compaction of not less than 35% and more preferably not less than 40%. Higher degrees of compaction indicate higher contents of fibrous material. Degree of compaction can be determined according to the following equation.

$$\text{Degree of compaction (\%)} = [\{(\text{tapped bulk density})$$

$$- (\text{loose bulk density})\}/(\text{tapped bulk density})] \times 100$$

As used herein, the term "loose bulk density" refers to a bulk density in a loosely packed state. This can be measured by providing a cylindrical vessel having a diameter of 5.03 cm and a height of 5.03 cm (and hence a capacity of 100 ml), introducing a sample uniformly into the vessel from a height of 23 cm while passing it through a JIS 22-mesh screen (710 $\mu$m), leveling the top surface of the sample, and then weighing it. Most typically, this can be measured by means of a Powder Tester (PT-D, manufactured by Hosokawa Micron Corp.).

The term "tapped bulk density" refers to a bulk density measured after the sample is closely packed by tapping.

Specifically, this can be measured as follows: After the loose bulk density of the sample is measured, a cap for exclusive use (an accessory to the Powder Tester manufactured by Hosokawa Micron Corp.) is attached to the top of the vessel. Then, the powder is added thereto until it reaches the upper end of the cap, and then tapped 180 times from a tapping height of 1.8 cm. After completion of the tapping, the cap is removed, the top surface of the powder is leveled at the upper end of the vessel, and the powder filling the 100 ml vessel is weighed.

Flowability index, which was proposed by Carr, is known to be an indicator of the flowability of a powder. This flowability index can be determined by measuring the angle of repose, angle of spatula, and degree of aggregation of the powder in addition to the aforesaid degree of compaction, calculating the respective indices from these four measured values, and summing them up. A detailed description thereof is given in "An Illustrated Explanation of Powder Properties (revised and enlarged edition)" (edited by the Japanese Society of Powder Technology and the Japanese Association of Powder Process Industry and Engineering, Nikkei Technical Books, 1985), page 151.

Usually, fibrous low-substituted hydroxypropyl cellulose having a high degree of compaction exhibits high binding power, but has low flowability. Consequently, it is difficult to utilize such low-substituted hydroxypropyl cellulose as a base material for dry direct tableting use. However, the product of the process of the present invention, which is obtained by impregnating low-substituted hydroxypropyl cellulose with a sugar or a sugar alcohol and then drying it, has good flowability.

A powder obtained simply by granulating low-substituted hydroxypropyl cellulose with the aid of water and drying the resulting granular material shows an improvement in flowability. However, this powder is reduced to finer particles as a result of shrinkage on drying. Moreover, this powder is reluctant to deformation in response to the force applied during tableting, thus showing a reduction in binding power. However, in the process of the present invention the product is obtained by impregnating low-substituted hydroxypropyl cellulose with a sugar or a sugar alcohol and then drying it, and the low-substituted hydroxypropyl cellulose is dried after the sugar or sugar alcohol is introduced into its interstices formed as a result of swelling by water. Consequently, it is believed that the shrinkage of the low-substituted hydroxypropyl cellulose on drying is suppressed. Moreover, owing to the presence of the interstitial sugar or sugar alcohol, the low-substituted hydroxypropyl cellulose easily deforms in response to the force applied during tableting and can hence retain its binding power.

The amount of sugar or sugar alcohol added is in the range of 30 to 100% by weight based on the low-substituted hydroxypropyl cellulose. If it is less than 30% by weight, a reduction in binding power due to shrinkage may not be suppressed when the low-substituted hydroxypropyl cellulose is moistened and then dried. If it is greater than 100% by weight, a reduction in binding power may result because of a decrease in low-substituted hydroxypropyl cellulose content.

The amount of water used during granulation is in the range of 200 to 600% by weight based on the low-substituted hydroxypropyl cellulose. It is preferable to use water by dissolving the sugar or sugar alcohol therein. The impregnation is carried out by dipping or the like.

After the wet granular material is dried, the dried granular material may optionally be pulverized and classified. No particular limitation is placed on the method for drying the wet granular material. For example, this may be done by drying the wet granular material at a temperature of about 60 to 80°C by means of a hot-air oven, or by drying it in the form of a fluidized bed having an intake temperature of about 60 to 80°C.

The product of the present invention, which is obtained by impregnating low-substituted hydroxypropyl cellulose with a sugar or a sugar alcohol and then drying it, preferably has a flowability index of not less than 60. If its flowability index is less than 60, the product may have such low flowability that the formation of tablets may be impossible because it may fail to flow out of the hopper of a tableting machine.

In addition to the low-substituted hydroxypropyl cellulose and the sugar or sugar alcohol, the base material for dry direct tableting in accordance with the present invention may further contain commonly used excipients such as lactose and corn starch; binders such as microcrystalline cellulose; disintegrators such as carboxymethylcellulose calcium and crosslinked carboxymethylcellulose sodium; and the like. In such a case, the finally obtained base material for dry direct tableting preferably has a flowability index of not less than 60.

The present invention is more specifically explained with reference to the following examples and comparative examples.

Example 1

An agitation granulator (Vertical Granulator FM-VG-05, manufactured by Powrex Corp.) having an internal volume of 5 liters was charged with 500 g of low-substituted hydroxypropyl cellulose (LH-11, manufactured by Shin-Etsu Chemical Co., Ltd.) containing 0.25 mole of hydroxypropoxyl substituent group and having a degree of compaction of 45%. While this low-substituted hydroxypropyl cellulose was agitated at a rotational speed of 800 rpm and a chopper speed of 900 rpm, 1,470 g of a 17 wt% aqueous solution of erythritol (i.e., 50% by weight of erythritol based on the low-substituted hydroxypropyl cellulose) was added thereto and a granulation process was then performed for 5 minutes.

The resulting granular material was dried in a hot-air oven at 80°C for a whole day and night. Thereafter, the dried granular material was pulverized with a small-sized pulverizer and then passed through a 80-mesh screen (with an opening of 177 $\mu$m) to obtain the desired product. The flowability index, binding power and disintegrability of the product thus obtained are shown in Table 1.

Example 2

An agitation granulator (Vertical Granulator FM-VG-05, manufactured by Powrex Corp.) having an internal volume of 5 liters was charged with 500 g of low-substituted hydroxypropyl cellulose containing 0.25 mole of hydroxypropoxyl substituent group and having a degree of compaction of 50%. While this low-substituted hydroxypropyl cellulose was agitated at a rotational speed of 800 rpm and a chopper speed of 900 rpm, 1,470 g of a 17 wt% aqueous solution of erythritol (i.e., 50% by weight of erythritol based on the low-substituted hydroxypropyl cellulose) was added thereto and a granulation process was then performed for 5 minutes.
The resulting granular material was dried in a hot-air oven at 80°C for a whole day and night. Thereafter, the dried granular material was pulverized with a small-sized pulverizer and then passed through a 80-mesh screen to obtain the desired product. The flowability index, binding power and disintegrability of the product thus obtained are shown in Table 1.

Example 3

An agitation granulator (Vertical Granulator FM-VG-05, manufactured by Powrex Corp.) having an internal volume of 5 liters was charged with 500 g of low-substituted hydroxypropyl cellulose (LH-11, manufactured by Shin-Etsu Chemical Co., Ltd.) containing 0.25 mole of hydroxypropoxyl substituent group and having a degree of compaction of 45%. While this low-substituted hydroxypropyl cellulose was agitated at a rotational speed of 800 rpm and a chopper speed of 900 rpm, 1,470 g of a 17 wt% aqueous solution of mannitol (i.e., 50% by weight of mannitol based on the low-substituted hydroxypropyl cellulose) was added thereto and a granulation process was then performed for 5 minutes.
The resulting granular material was dried in a hot-air oven at 80°C for a whole day and night. Thereafter, the dried granular material was pulverized with a small-sized pulverizer and then passed through a 80-mesh screen to obtain the desired product. The flowability index, binding power and disintegrability of the product thus obtained are shown in Table 1.

Example 4

An agitation granulator (Vertical Granulator FM-VG-05, manufactured by Powrex Corp.) having an internal volume of 5 liters was charged with 500 g of low-substituted hydroxypropyl cellulose (LH-11, manufactured by Shin-Etsu Chemical Co., Ltd.) containing 0.25 mole of hydroxypropoxyl substituent group and having a degree of compaction of 45%. While this low-substituted hydroxypropyl cellulose was agitated at a rotational speed of 800 rpm and a chopper speed of 900 rpm, 1,470 g of a 17 wt% aqueous solution of sorbitol (i.e., 50% by weight of sorbitol based on the low-substituted hydroxypropyl cellulose) was added thereto and a granulation process was then performed for 5 minutes.
The resulting granular material was dried in a hot-air oven at 80°C for a whole day and night. Thereafter, the dried granular material was pulverized with a small-sized pulverizer and then passed through a 80-mesh screen to obtain the desired product. The flowability index, binding power and disintegrability of the product thus obtained are shown in Table 1.

Comparative Example 1

An agitation granulator (Vertical Granulator FM-VG-05, manufactured by Powrex Corp.) having an internal volume of 5 liters was charged with 500 g of low-substituted hydroxypropyl cellulose (LH-11, manufactured by Shin-Etsu Chemical Co., Ltd.) containing 0.25 mole of hydroxypropoxyl substituent group and having a degree of compaction of 45%. While this low-substituted hydroxypropyl cellulose was agitated at a rotational speed of 800 rpm and a chopper speed of 900 rpm, 1,220 g of water (i.e., the same amount of water as used in Example 1) was added thereto and a granulation process was then performed for 5 minutes.
The resulting granular material was dried in a hot-air oven at 80°C for a whole day and night. Thereafter, the dried granular material was pulverized with a small-sized pulverizer and then passed through a 80-mesh screen to obtain the desired product. The flowability index, binding power and disintegrability of the product thus obtained are shown in Table 1.
The procedures for evaluation tests were as follows:

Flowability index

This was determined by measuring the degree of compaction, angle of repose, angle of spatula, and degree of aggregation of each product with a Powder Tester (manufactured by Hosokawa Micron Corp.), and summing up the indices derived from these values.

Binding power

A 200-mg sample of each product was weighed out. Using an IR tableting machine, a tablet having a diameter of 10 mm was formed by pressing the sample at 9.8 MPa for 30 seconds. The hardness of this tablet was measured.

Disintegrability

According to the disintegration test method described in the Pharmacopoeia of Japan (13th Edition), the disintegration time of each product was measured with a test fluid comprising water at 37°C.

The test results obtained according to the above-described procedures are shown in Table 1. For purposes of reference, the flowability index, binding power and disintegrability of low-substituted hydroxypropyl cellulose (LH-11, manufactured by Shin-Etsu Chemical Co., Ltd.) used in Example 1 are also shown in Table 1.

Table 1

| | Base material | | | Evaluation | | |
|---|---|---|---|---|---|---|
| | Degree of compaction of LHPC (%) | Sugar or sugar alcohol | | Flowability index | Binding power (N) | Disintegrability (min.) |
| | | Type | Amount based on LHPC (wt.%) | | | |
| Example 1 | 45 | Erythritol | 50 | 67 | 222 | 4.3 |
| Example 2 | 50 | Erythritol | 50 | 61 | 350 | 5.4 |
| Example 3 | 45 | Mannitol | 50 | 66 | 170 | 2.3 |
| Example 4 | 45 | Sorbitol | 50 | 65 | 185 | 3.3 |
| Comparative Example 1 | 45 | None | 0 | 62 | 69 | 0.5 |
| LH-11 | 45 | - | - | 45 | 175 | 8.9 |
| *"LHPC" stands for low-substituted hydroxypropyl cellulose. | | | | | | |

From the above-described results, it can be seen that a product obtained by impregnating low-substituted hydroxypropyl cellulose with a sugar or a sugar alcohol and then drying it is a powder having high binding power and good flowability. This powder also has excellent disintegration properties and can hence be utilized as a base material for dry direct tableting.

**Claims**

1.  A method of manufacturing a base material for dry direct tableting comprising impregnating particles of low-substituted hydroxypropyl cellulose having an hydroxypropyl content in the range 5.0 to 16.0% by weight with a sugar or sugar alcohol such that 30 to 100% by weight sugar or sugar alcohol is added based on said low-substituted hydroxypropyl cellulose, comprising the steps:

    (a) dry blending low-substituted hydroxypropyl cellulose and a sugar or sugar alcohol;
    (b) adding 200 to 600% by weight of water based on said low-substituted hydroxypropyl cellulose to the resulting blend while agitating it; and
    (c) drying.

2.  A method of manufacturing a base material for dry direct tableting comprising impregnating particles of low-substituted hydroxypropyl cellulose having an hydroxypropyl content in the range 5.0 to 16.0% by weight with a sugar or sugar alcohol such that 30 to 100% by weight sugar or sugar alcohol is added based on said low-substituted hydroxypropyl cellulose; comprising the steps:

(a) agitating low-substituted hydroxypropyl cellulose;
(b) adding an aqueous solution of a sugar or sugar alcohol; and
(c) drying.

3. The method of either of claims 1 or 2 wherein said sugar or sugar alcohol is one or more compounds selected from the group consisting of erythritol, mannitol and sorbitol.

**Patentansprüche**

1. Verfahren zur Herstellung eines Ausgangsmaterials für die trockene Direkttablettierung, umfassend das Imprägnieren von Teilchen aus niedrigsubstituierter Hydroxypropylcellulose, die einen Hydroxypropylgehalt im Bereich von 5,0 bis 16,0 Gew.-% aufweist, mit einem Zucker oder Zuckeralkohol, so daß, bezogen auf die niedrigsubstituierte Hydroxypropylcellulose, 30 bis 100 Gew.-% Zucker oder Zuckeralkohol zugegeben werden, mit den Schritten:

(a) Trockenes Vermischen von niedrigsubstituierter Hydroxypropylcellulose und einem Zucker oder Zuckeralkohol,
(b) Zugeben von 200 bis 600 Gew.-% Wasser, bezogen auf die niedrigsubstituierte Hydroxypropylcellulose, zu der resultierenden Mischung unter Rühren und
(c) Trocknen.

2. Verfahren zur Herstellung eines Ausgangsmaterials für die trockene Direkttablettierung, umfassend das Imprägnieren von Teilchen aus niedrigsubstituierter Hydroxypropylcellulose, die einen Hydroxypropylgehalt im Bereich von 5,0 bis 16,0 Gew.-% aufweist, mit einem Zucker oder Zuckeralkohol, so daß, bezogen auf die niedrigsubstituierte Hydroxypropylcellulose, 30 bis 100 Gew.-% Zucker oder Zuckeralkohol zugegeben werden, mit den Schritten:

(a) Rühren von niedrigsubstituierter Hydroxypropylcellulose,
(b) Zugeben einer wäßrigen Lösung eines Zuckers oder Zuckeralkohols und
(c) Trocknen.

3. Verfahren nach Anspruch 1 oder 2, wobei es sich bei dem Zucker oder Zuckeralkohol um eine oder mehrere Verbindungen handelt, ausgewählt aus der Gruppe bestehend aus Erythritol, Mannitol und Sorbitol.

**Revendications**

1. Méthode de fabrication d'un excipient pour compression directe à sec comprenant l'imprégnation de particules d'hydroxypropylcellulose à substitution basse, la quantité d'hydroxypropyl étant comprise entre 5.0 à 16.0 % du poids avec un sucre ou polyol de façon telle que 30 à 100 % du poids constitué de sucre ou polyol est ajouté à l'hydroxypropylcellulose à substitution basse ; comprenant les étapes suivantes :

(a) Mélanger à sec d'hydroxypropylcellulose à substitution basse et d'un sucre ou polyol ;
(b) Ajouter de l'eau représentant de 200 à 600 % du poids de ladite hydroxypropylcellulose à substitution basse au mélange obtenu durant son agitation ; et
(c) Sécher.

2. Méthode de fabrication d'un excipient pour compression directe à sec, comprenant l'imprégnation de particules d'hydroxypropylcellulose à substitution basse, la quantité d'hydroxypropyl étant comprise entre 5.0 à 16.0 % du poids avec un sucre ou polyol de façon telle que 30 à 100 % du poids constitué de sucre ou polyol est ajouté à l'hydroxypropylcellulose à substitution basse ; comprenant les étapes suivantes :

(a) Agitation d'hydroxypropylcellulose à substitution basse
(b) Adjonction d'une solution aqueuse de sucre ou polyol ; et
(c) séchage.

3. Méthode des revendications 1 ou 2, **caractérisée en ce que** ledit sucre ou polyol représente un ou plusieurs composés sélectionnés dans le groupe constitué de l'erythritol, mannitol et sorbitol.

## REFERENCES CITED IN THE DESCRIPTION

**Patent documents cited in the description**

- JP 464279271 B
- JP 575310082 B
- EP 0957112 A
- JP 732410195 B

**Non-patent literature cited in the description**

- An Illustrated Explanation of Powder Properties (revised and enlarged edition. 1985, 151